# EUROPEAN PATENT APPLICATION

(11) **EP 3 828 721 A2**
(43) Date of publication of application: **02.06.2021**
(21) Application number: 21165228.4
(22) Date of filing: 26.03.2021
(51) Int. Cl.: G06F 16/00

(54) **METHOD AND APPARATUS FOR CONVERTING UNDIRECTED RELATIONSHIP TO DIRECTED RELATIONSHIP, DEVICE, STORAGE MEDIUM, AND COMPUTER PROGRAM PRODUCT**

(30) Priority: 09.09.2020 CN 202010938312
(71) Applicant: Beijing Baidu Netcom Science and Technology Co., Ltd., Beijing 100085 (CN)
(72) Inventor: WU, Yiling, Beijing, Beijing 100085 (CN); LIU, Yan, Beijing, Beijing 100085 (CN); WANG, Bing, Beijing, Beijing 100085 (CN)
(74) Representative: Müller, Christian Stefan Gerd

(57) **Abstract**

A method and apparatus for converting an undirected relationship to a directed relationship, device, storage medium and a computer program product are provided. An implementation of the method includes: determining a graph of undirected relationships between a plurality of subjects based on time characteristics of co-occurrences among the plurality of subjects; acquiring time characteristic intervals of edges between an individual subject and related subjects thereof from the graph of the undirected relationships; sorting the time characteristic intervals, and traversing the time characteristic intervals according to a sorted order, to find a target time characteristic interval not overlapping with a traversed time characteristic interval; and obtaining a directed one-way edge of the individual subject, by using a related subject corresponding to the target time characteristic interval as an end vertex of the one-way edge and using the individual subject as a start vertex of the one-way edge.

## Description

### TECHNICAL FIELD

Embodiments of the present disclosure relate to the field of computer technology, particularly to the technology field of big data, and more particularly to a method and apparatus for converting an undirected relationship to a directed relationship, a device, a storage medium and a computer program product.

### BACKGROUND

There are various kinds of relationship data in real life. For example, the co-occurrence relationship between the ones traveling together. Sine two related persons show up concurrently, it seems like involving an undirected relationship. However, when combined with specific scenarios, such as the transmission of messages or the spread of diseases, the person who travels with the source of the message or diseases later is impossible to transmit the message or viruses to the person of who travels with the source of the message or diseases earlier. Therefore, in this particular spread scenario, the use of the graph of undirected relationship cannot help to analyze the spear or transmission.

### SUMMARY

In order to solve one or more technical problems mentioned above, embodiments of the present disclosure provides a method and apparatus for converting an undirected relationship to a directed relationship, device and storage medium.

In a first aspect, some embodiments of the present disclosure provide a method for converting an undirected relationship to a directed relationship, the method includes: determining a graph of undirected relationships between a plurality of subjects based on time characteristics of co-occurrences among the plurality of subjects; acquiring time characteristic intervals of edges between an individual subject and related subjects thereof from the graph of the undirected relationships, wherein a time characteristic interval of an edge represents that the individual subject and a related subject co-occurred within the time characteristic interval; sorting the time characteristic intervals, and traversing the time characteristic intervals according to a sorted order, to find a target time characteristic interval not overlapping with a traversed time characteristic interval; and obtaining a directed one-way edge of the individual subject, by using a related subject corresponding to the target time characteristic interval as an end vertex of the one-way edge and using the individual subject as a start vertex of the one-way edge.

In a second aspect, some embodiments of the present disclosure provide an apparatus for converting an undirected relationship to a directed relationship, the apparatus includes: a determination module, configured to determine a graph of undirected relationships between a plurality of subjects based on time characteristics of co-occurrences among the plurality of subjects; an acquisition module, configured to acquire time characteristic intervals of edges between an individual subject and related subjects thereof from the graph of the undirected relationships, where a time characteristic interval of an edge represents that the individual subject and a related subject co-occurred within the time characteristic interval; a searching module, configured to sort the time characteristic intervals, and traversing the time characteristic intervals according to a sorted order, to find a target time characteristic interval not overlapping with a traversed time characteristic interval; and a conversion module, configured to obtain a directed one-way edge of the individual subject, by using a related subject corresponding to the target time characteristic interval as an end vertex of the one-way edge and using the individual subject as a start vertex of the one-way edge.

In a third aspect, some embodiments of the present disclosure provide an electronic device, the electronic device includes: at least one processor; and a memory, in communication with the at least one processor; where, the memory stores instructions executable by the at least one processor to enable the at least one processor to perform the method according to any one of the implementations described in the first aspect.

In a fourth aspect, some embodiments of the present disclosure provide a non-transitory computer-readable storage medium having stored thereon computer instructions executable for causing the computer to perform the method according to any one of the implementations described in the first aspect.

In a fifth aspect, some embodiments of the present disclosure provide a computer program product. The computer program product includes a computer program. the computer program, when executed by a processor, implementing the method according to any one of the implementations described in the first aspect.

It should be understood that the content described in this section is not intended to identify the key or important features of embodiments of the present disclosure, nor is it intended to limit the scope of the present disclosure. Other features of the present disclosure will become easily understood by the following description.

### BRIEF DESCRIPTION OF THE DRAWINGS

Other features, objectives and advantages of the present disclosure will become more apparent upon reading the detailed description to non-limiting embodiments with reference to the accompanying drawings. The accompanying drawings are used to better understand the present solution and do not constitute a limitation to the present disclosure, in which:
FIG. 1 is an exemplary system architecture that embodiments of the present disclosure may be applied thereto;
FIG. 2 is a flow of a method for converting an undirected relationship to a directed relationship according to an embodiment of the present disclosure;
FIG. 3 is a schematic structural diagram of a graph of undirected relationships according to an embodiment of the present disclosure;
FIG. 4 is a schematic flow of a method for sorting time characteristic intervals according to an embodiment of the present disclosure;
FIG. 5 is a structural diagram of a one-way edge according to an embodiment of the present disclosure;
FIG. 6 is a schematic flow of method for converting an undirected relationship to a directed relationship according to another embodiment of the present disclosure;
FIG. 7 is a structural diagram of a two-way edge according to an embodiment of the present disclosure;
FIG. 8 is a schematic structural diagram of an apparatus for converting an undirected relationship to a directed relationship according to an embodiment of the present disclosure;
FIG. 9 is a schematic structural diagram of an electronic device for converting an undirected relationship to a directed relationship according to an embodiment of the present disclosure.

### DETAILED DESCRIPTION OF EMBODIMENTS

Embodiments of present disclosure will be described below in detail with reference to the accompanying drawings. It should be appreciated that the specific embodiments described herein are merely used for explaining the relevant disclosure, rather than limiting the disclosure. In addition, it should be noted that, for the ease of description, only the parts related to the relevant disclosure are shown in the accompanying drawings.

It should also be noted that some embodiments in the present disclosure and some features in the disclosure may be combined with each other on a non-conflict basis. Features of the present disclosure will be described below in detail with reference to the accompanying drawings and in combination with embodiments.

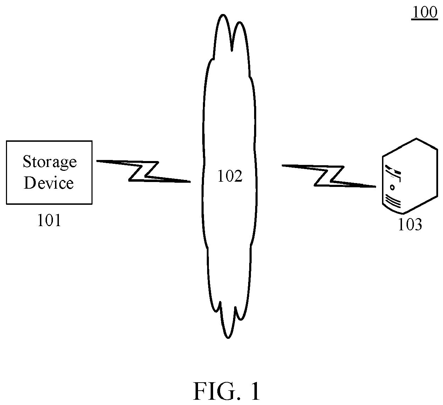

FIG. 1 illustrates an exemplary system architecture 100 of a method or an apparatus for converting an undirected relationship to a directed relationship in which an embodiment of the present disclosure may be implemented.

As shown in FIG. 1, the system architecture 100 may include a storage device 101, a network 102, and a server 103. The network 102 is used to provide a communication link medium between the storage device 101 and the server 103. The network 102 may include various types of connections, such as wired, wireless communication links, or optic fibers.

The storage device 101 may interact with the server 130 via the network 102. Data of undirected relationships may be provided in the storage device 101 including but not limited to database, user terminal, etc..

The server 103 may be a server that provides various services. For example, the server may analyze the data of undirected relationships acquired from the storage device 101, to generated an processing result (such as converting the data of undirected relationships to data of directed relationships).

It should be noted that the server 103 may be hardware or software. When the server 103 is hardware, it may be implemented as a distributed server cluster composed of a plurality of servers, or as an individual server. When the server 103 is software, it may be implemented as a plurality of software or software modules (for example, for providing distributed services), or as an individual software or software module, which is not specifically limited herein.

It should be noted that the method for converting an undirected relationship to a directed relationship provided in embodiments of the present disclosure may be performed by the server 103. Accordingly, the apparatus for recognizing face-swap may be provided in the server 103.

It should be understood that the number of terminal devices, networks, and servers in Fig. 1 is merely illustrative. Depending on the implementation needs, there may be any number of terminal devices, networks, and servers.

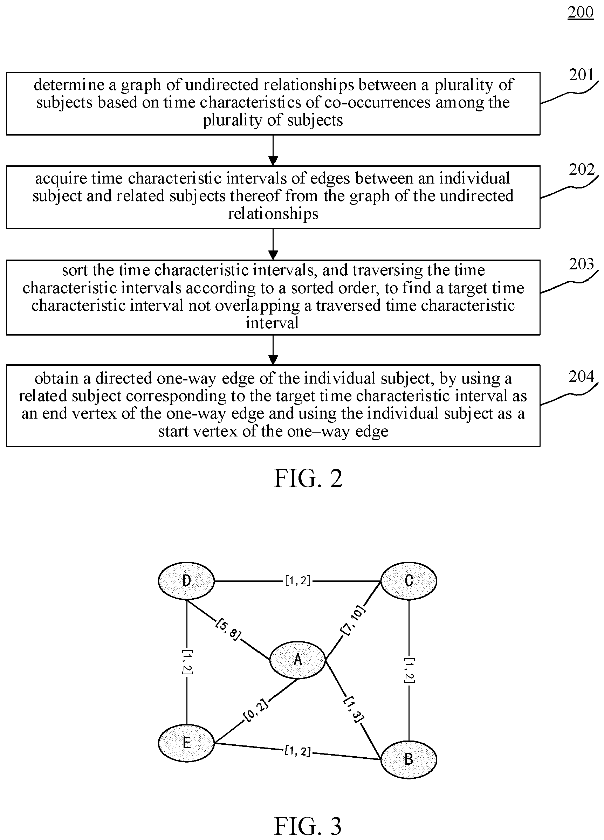

With further reference to FIG. 2, illustrating a flow 200 of a method for converting an undirected relationship to a directed relationship according to an embodiment of the present disclosure. The method for converting an undirected relationship to a directed relationship includes the following steps:

Step 201, determining a graph of undirected relationships between a plurality of subjects based on time characteristics of co-occurrences among the plurality of subjects traveling together.

In an embodiment, the execution body (for example, the server 103 shown in FIG. 1) of the method for converting an undirected relationship to a directed relationship may determine a graph of undirected relationships between a plurality of subjects based on time characteristics of co-occurrences among the plurality of subjects traveling together. Co-occurrence refers to the simultaneous occurrence of two related subjects. For example, the co-occurrence relationship between persons, since two persons related to each other show up at the same time, the relationship between the two persons is undirected. In the field of disease transmission, the contact relationship between two infected persons can be considered as a co-occurrence relationship between the two infected persons. Taking the tracing back to an infection source under the global epidemic as an example, data that can help trace back to an infection source include: a person who close contacts of an infection person and the contact time, track information of an infection person collected by a mobile network operator, Bluetooth connection data of an infection person and other persons, and the like. These data are often undirected relationship data with temporal characteristics. For example, the subject A, B, C, D and E are all infected with a disease, the subject A and the subject B are in contact on a certain day at 13:00-15:00, the subject A and the subject C are in contact on the same day at 19:00-22:00, the subject A and the subject D are in contact on the same day at 17:00-20:00, the subject A and the subject E are in contact on the same day at 12:00-14:00, the subject D and the subject E are in contact on the same day at 13:00-14:00, the subject B and the subject E are in contact on the same day at 13:00-14:00, the subject B and the subject C are in contact on the same day at 13:00-14:00, and the subject D and the subject C are in contact on the same day at 13:00-14:00. As shown in FIG. 3, the above-described execution body may generate a graph of undirected relationships between the subjects A, B, C, D, and E based on the time characteristics of the co-occurrence relationships between the subjects A, B, C, D, and E.

In some alternative implementations of the present embodiment, the time characteristics of co-occurrences between a plurality of subjects may be determined based on the time of the earliest contact and the time of the most recent contact among the plurality of subjects. For example, if the time of the earliest contact between the subject A and the subject B is on a certain day at 13:00, and the time of the latest contact is on the same day at 15:00. So that, based on the time of the earliest contact and the time of the latest contact between the subject A and the subject B, the time characteristic intervals [1, 3] of the co-occurrence of the subject A and the subject B can be generated using mapping relationship.

Step 202, acquiring time characteristic intervals of edges between an individual subject and related subjects thereof from the graph of the undirected relationships.

In the present embodiment, the execution body may obtain the time characteristic intervals of edges between an individual subject and related subjects thereof from the graph of the undirected relationships. Taking the relationship diagram shown in FIG. 3 as an example, all the subjects B, C, D, and E having a co-occurrence relationship with the subject A may be selected as the related subjects of the subject A, and the time characteristic intervals [0, 2], [1, 3], [7, 10], [5, 8] of the edges formed by the subject A and the related subjects B, C, D, and E may be acquired. A time characteristic interval of an edge represents that an individual subject and a relational subject have a co-occurrence relationship within the time characteristic interval.

Step 203: sorting the time characteristic intervals, and traversing the time characteristic intervals according to a sorted order, to find a target time characteristic interval not overlapping a traversed time characteristic interval.

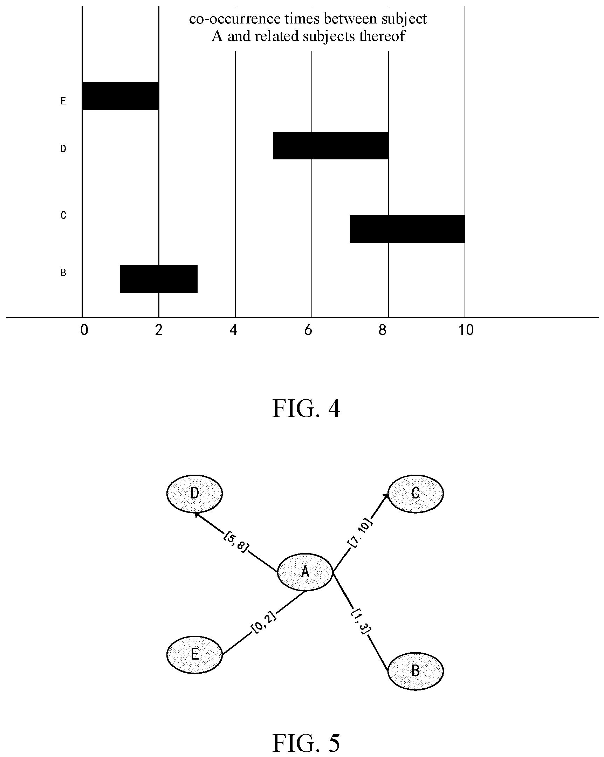

In an embodiment, the execution body may sort the time characteristic intervals, traverse the time characteristic intervals according to the sorted order, to find the target time characteristic interval not overlapping the traversed time characteristic intervals. Taking the graph of relationships shown in FIG. 3 as an example, the time characteristic intervals [0, 2], [1, 3], [7, 10], [5, 8] obtained according to step 202 are sorted in chronological order, and the sorted result is shown in FIG. 4. Thereafter, the corresponding time characteristic intervals [0, 2], [1, 3], [5, 8], [7, 10] are traversed in the order E-B-D-C. When traversing the time characteristic interval [5, 8] corresponding to D, a first target time characteristic interval, the first one that does not overlap with the traversed time characteristic intervals [0, 2], [1, 3], is found. Similarly, when traversing the time characteristic interval [7, 10] corresponding to C, a second target time characteristic interval, the second one that does not overlap with the traversed time characteristic intervals [0, 2], [1, 3], is found.

Step 204, obtaining a directed one-way edge of the individual subject, by using a related subject corresponding to the target time characteristic interval as an end vertex of the one-way edge and using the individual subject as a start vertex of the one-way edge.

In an embodiment, the execution body may use a related subject corresponding to the target time characteristic interval as the end vertex of a one-way edge, and use the individual subject as the start vertex of the one-way edge, to obtain a directed one-way edge of the individual subject. Still taking the graph of relationships shown in FIG. 3 as an example, it can be seen from FIG. 4 that the co-occurrence times between A and B, E is earlier, the co-occurrence times between A and C, D is later, and there is no overlap therebetween. Without considering the presence of other infection sources, the whole infection process may be A, B, E are infected first, and then C, D are infected thereafter. Particularly, it can only be that A infects C, A infects D, but cannot be that C infects A, or C infects D. That is, A infects C, or A infects D, can be represented by a directed one-way edge between A and C, or between A and D. As shown in FIG. 5, a directed one-way edge is obtained by taking A as the start vertex of the one-way edge and C or D as the end vertex of the one-way edge.

Compared with prior arts, the embodiment of the present disclosure provides a unified method for converting a large amount of undirected relationship data to directed relationship data, thereby saving labor cost. By converting the undirected relationship data having time attributes into directed relationship data, it is possible to more conveniently and accurately trace the infection path of infected persons, thereby reducing the time for tracing the infection source.

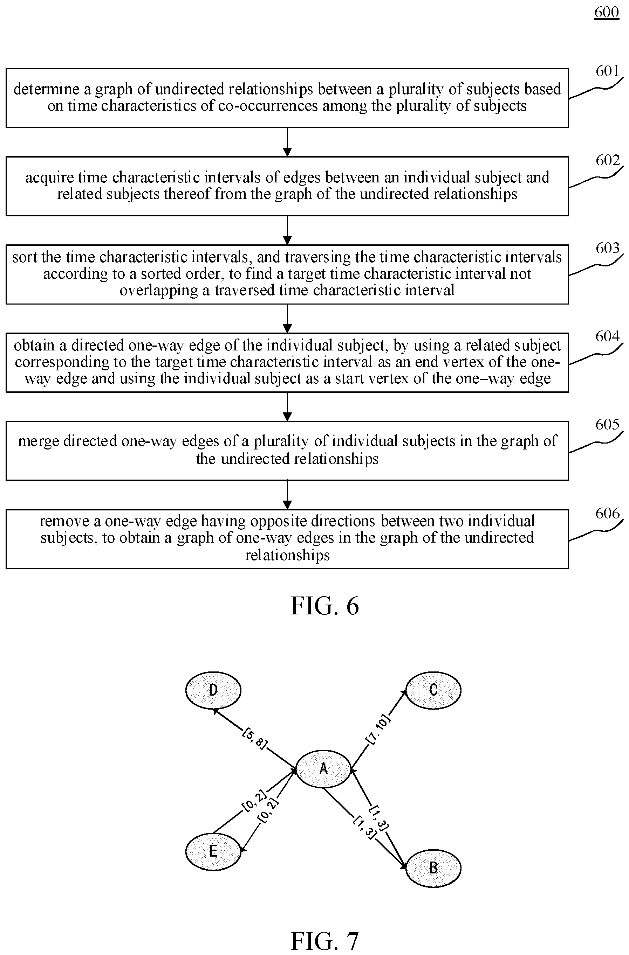

Referring further to FIG. 6, there is shown a flow chart of the method for converting undirected relationships to directed relationships according to another embodiment of the present disclosure, in which the processing details of steps 601-604 and the technical effect thereof are the same as that of steps 201-204 described in FIG. 2, and details are not described herein. The method for converting undirected relationships to directed relationships further comprises:

Step 605, merging directed one-way edges of a plurality of individual subjects in the graph of the undirected relationships.

In an embodiment, the execution body (for example, the server 103 shown in FIG. 1) of the method for converting undirected relationship to directed relationship may merge the directed one-way edges of a plurality of individual subjects in the graph of undirected relationships. Here, steps 201-204 described in FIG. 2 may be performed for each of the plurality of individual subjects, resulting in one-way edges of each individual subject. For example, in the graph of relationships shown in FIG. 3, in addition to acquiring one-way edges of the subject A, the one-way edges of each of the subjects B, C, D, and E can be obtained in the same manner. Thereafter, the directed one-way edges of each subject are merged.

Step 606, removing a one-way edge having opposite directions between two individual subjects, to obtain a graph of one-way edges in the graph of the undirected relationships.

In the present embodiment, the execution body can remove the one-way edges having opposite directions between the two subjects to obtain the graph of one-way relationships of the graph of undirected relationships. The one-way edge having opposite directions refers to an edge with two subjects as start vertex and end vertex mutually. For example, as shown in FIG. 7, there are a two-way edge between the subject A and subject E and a two-way edge between the subject A and subject B, where the two-way edge is formed by two one-way edges in opposite directions. In the present embodiment, by removing the two one-way edges having opposite directions obtained from the graph of undirected relationships, all of the one-way edges in the graph of undirected relationships can be obtained.

In some alternative implementations of the present embodiment, if there is no one-way edge between a individual subject and a related subject thereof in the graph of undirected relationships, the edge between the individual subject and the related subject thereof is marked as two-way edge, so that a graph of two-way edges of the graph of undirected relationships is obtained. With the graph of relationships shown in FIG. 3 as an example, by performing steps 201-204 in FIG. 2, it can be seen that although in the entire infection process it is only possible that A, B, E are infected first and C, D are infected later, the infection direction between A, B and A, E cannot be determined. That is, there is no one-way edge relationship between A, B, and between A, E. Then, the A, B and A, E can be marked as two-way edges, as shown in FIG. 7. By determining the two-way edges in the graph of undirected relationships, it is possible to distinguish the information having the direction of transmission from the information not having the direction of transmission, thereby facilitating efficient in tracing the infection source.

An embodiment of the present disclosure also provides a method for converting the undirected relationship data into the directed relationship data. That is, determining a graph of directed edges between individual subjects and related subjects thereof in the graph of the undirected relationships, based on SPARK-GraphX by using an aggregate function of GraphX, where the graph of directed edges includes a one-way edge and a two-way edge. A digraph is determined by using the aggregation function of GraphX, and the pseudo code is as follows:
a) Val graph = Graph.fromEdges/*constructing a graph based on undirected relationships*/
b) Val vertices_with_neighbouredges = graph.aggregateMessages/*merging information of related edges at each vertex*/
c) Val vertices_get_out_vertices = vertices_with_neighbouredges.mapValues/*determining a local directed edge of each vertex, based on the merged information of vertex point to local information*/
d) Val oneside_edges = vertices_get_out_vertices/* (vid, (dstid list with vid as the starting vertex) */
   - flatMapValues/* tile */
   - map.reduceByKey.filter.map/* merging all one-way edges to remove edges which is locally one-way but globally two-way*/
e) Val all_sorted_edges = edges.map/*sorting full amount of edges*/
f) Val twoside_edges_sorted = all_sorted_edges.subtractByKey (oneside_edges.map)/*sorted two-way edges in full edges */
g) Val all_directed_edges = twoside_edges_sorted.union (twoside_edges_sorted.map).union (oneside_edges)/* union opposite directions in two-way edges and one-way edge*/

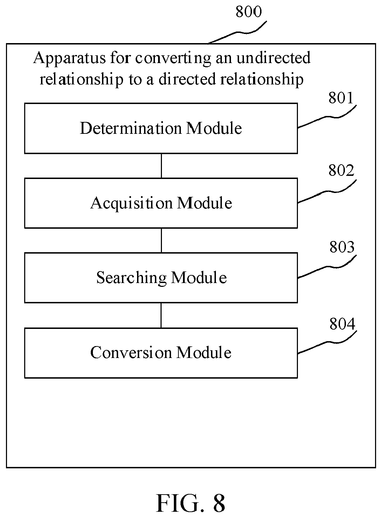

With further reference to FIG. 8, as an implementation of the method shown in above figures, embodiments of the present disclosure provide an apparatus for converting an undirected relationship to a directed relationship. The apparatus embodiments are corresponding to the method embodiments shown in FIG. 2, and the apparatus particularly may be applied to various electronic devices.

As shown in FIG. 8, the apparatus 800 for converting an undirected relationship to a directed relationship may include: a determination module 801, an acquisition module 802, a searching module 803, and a conversion module 804. The determination module 801 is configured to determine a graph of undirected relationships between a plurality of subjects based on time characteristics of co-occurrences among the plurality of subjects; the acquisition module 802 is configured to acquire time characteristic intervals of edges between an individual subject and related subjects thereof from the graph of the undirected relationships, where a time characteristic interval of an edge represents that the individual subject and a related subject co-occurred within the time characteristic interval; the searching module 803 is configured to sort the time characteristic intervals, and traversing the time characteristic intervals according to a sorted order, to find a target time characteristic interval not overlapping with a traversed time characteristic interval; and the conversion module 804 is configured to obtain a directed one-way edge of the individual subject, by using a related subject corresponding to the target time characteristic interval as an end vertex of the one-way edge and using the individual subject as a start vertex of the one-way edge.

In this embodiment, the processing details of the determination module 801, the acquisition module 802, the search module 803, and the conversion module 804 in the apparatus 800 for converting an undirected relationship to a directed relationship, and the technical effects thereof can be referred to the relevant descriptions of steps 201-204 in the corresponding method embodiments illustrated in Fig. 2, and will not be repeated here.

In some alternative implementations of the present embodiment, the apparatus for converting an undirected relationship to a directed relationship further includes: a merging module, configured to merge directed one-way edges of a plurality of individual subjects in the graph of the undirected relationships; and a one-way graph module, configured to remove one-way edges having opposite directions between two individual subjects, to obtain a graph of one-way edges of the graph of the undirected relationships.

In some alternative implementations of the present embodiment, the apparatus further includes: a two-way graph module, configured to: mark an edge between the individual subject and the related subject thereof as a two-way edge, to obtain a graph of two-way edges of the graph of the undirected relationships, in response to there being no one-way edge between an individual subject and a related subject thereof in the graph of the undirected relationships.

In some alternative implementations of the present embodiment, the apparatus is further configured to: determine a graph of directed edges between individual subjects and related subjects thereof in the graph of the undirected relationships, based on SPARK-GraphX by using an aggregate function of GraphX, where the graph of directed edges includes a one-way edge and a two-way edge.

In some alternative implementations of the present embodiment, the apparatus further includes: a relationship determination module, configured to determine the time characteristics of the co-occurrences among the plurality of subjects based on the time of the earliest co-occurrence and the time of the most recent co-occurrence.

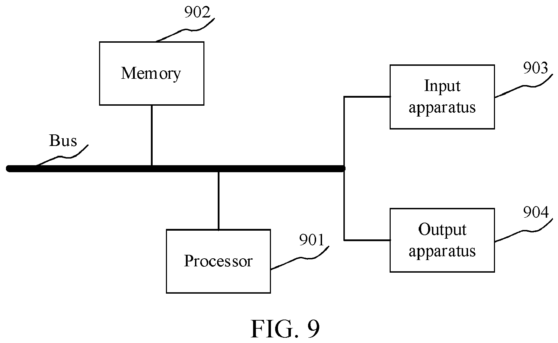

As shown in Fig. 9, which is a block diagram of an electronic device of a method for converting an undirected relationship to a directed relationship according to an embodiment of the present disclosure. The electronic device is intended to represent various forms of digital computers, such as laptop computers, desktop computers, workbenches, personal digital assistants, servers, blade servers, mainframe computers, and other suitable computers. The electronic device may also represent various forms of mobile apparatuses, such as personal digital processing, cellular phones, smart phones, wearable devices, and other similar computing apparatuses. The components shown herein, their connections and relationships, and their functions are merely examples, and are not intended to limit the implementation of the present disclosure described and/or claimed herein.

As shown in Fig. 9, the electronic device includes: one or more processors 901, a memory 902, and interfaces for connecting various components, including high-speed interfaces and low-speed interfaces. The various components are connected to each other using different buses, and may be installed on a common motherboard or in other methods as needed. The processor may process instructions executed within the electronic device, including instructions stored in or on the memory to display graphic information of GUI on an external input/output apparatus (such as a display device coupled to the interface). In other embodiments, a plurality of processors and/or a plurality of buses may be used together with a plurality of memories if desired. Similarly, a plurality of electronic devices may be connected, and the devices provide some necessary operations (for example, as a server array, a set of blade servers, or a multi-processor system). In Fig. 9, one processor 901 is used as an example.

The memory 902 is a non-transitory computer readable storage medium provided by embodiments of the present disclosure. The memory stores instructions executable by at least one processor, so that the at least one processor performs the method for converting an undirected relationship to a directed relationship provided by embodiments of the present disclosure. The non-transitory computer readable storage medium of the present disclosure stores computer instructions for causing a computer to perform the method for converting an undirected relationship to a directed relationship provided by the present disclosure.

The memory 902, as a non-transitory computer readable storage medium, may be used to store non-transitory software programs, non-transitory computer executable programs and modules, such as program instructions/modules corresponding to the method for converting an undirected relationship to a directed relationship in embodiments of the present disclosure (for example, the determination module 801, the acquisition module 802, the searching module 803, and the conversion module 804 shown in Fig. 8). The processor 901 executes the non-transitory software programs, instructions, and modules stored in the memory 902 to execute various functional applications and data processing of the server, that is, to implement the method for converting an undirected relationship to a directed relationship in the foregoing method embodiments.

The memory 902 may include a storage program area and a storage data area, where the storage program area may store an operating system and at least one function required application program; and the storage data area may store data created by the use of the electronic device according to the method for converting an undirected relationship to a directed relationship, etc. In addition, the memory 902 may include a high-speed random access memory, and may also include a non-transitory memory, such as at least one magnetic disk storage device, a flash memory device, or other non-transitory solid-state storage devices. In some embodiments, the memory 902 may optionally include memories remotely provided with respect to the processor 901, and these remote memories may be connected to the electronic device of the method for converting an undirected relationship to a directed relationship through a network. Examples of the above network include but are not limited to the Internet, intranet, local area network, mobile communication network, and combinations thereof.

The electronic device of the method for converting an undirected relationship to a directed relationship may further include: an input apparatus 903 and an output apparatus 904. The processor 901, the memory 902, the input apparatus 903, and the output apparatus 904 may be connected through a bus or in other methods. In Fig. 9, connection through a bus is used as an example.

The input apparatus 903 may receive input digital or character information, and generate key signal inputs related to user settings and function control of the electronic device of the method for converting an undirected relationship to a directed relationship, such as touch screen, keypad, mouse, trackpad, touchpad, pointing stick, one or more mouse buttons, trackball, joystick and other input apparatuses. The output apparatus 904 may include a display device, an auxiliary lighting apparatus (for example, LED), a tactile feedback apparatus (for example, a vibration motor), and the like. The display device may include, but is not limited to, a liquid crystal display (LCD), a light emitting diode (LED) display, and a plasma display. In some embodiments, the display device may be a touch screen.

Various embodiments of the systems and technologies described herein may be implemented in digital electronic circuit systems, integrated circuit systems, dedicated ASICs (application specific integrated circuits), computer hardware, firmware, software, and/or combinations thereof. These various embodiments may include: being implemented in one or more computer programs that can be executed and/or interpreted on a programmable system that includes at least one programmable processor. The programmable processor may be a dedicated or general-purpose programmable processor, and may receive data and instructions from a storage system, at least one input apparatus, and at least one output apparatus, and transmit the data and instructions to the storage system, the at least one input apparatus, and the at least one output apparatus.

These computing programs (also referred to as programs, software, software applications, or codes) include machine instructions of the programmable processor and may use high-level processes and/or object-oriented programming languages, and/or assembly/machine languages to implement these computing programs. As used herein, the terms "machine readable medium" and "computer readable medium" refer to any computer program product, device, and/or apparatus (for example, magnetic disk, optical disk, memory, programmable logic apparatus (PLD)) used to provide machine instructions and/or data to the programmable processor, including machine readable medium that receives machine instructions as machine readable signals. The term "machine readable signal" refers to any signal used to provide machine instructions and/or data to the programmable processor.

In order to provide interaction with a user, the systems and technologies described herein may be implemented on a computer, the computer has: a display apparatus for displaying information to the user (for example, CRT (cathode ray tube) or LCD (liquid crystal display) monitor); and a keyboard and a pointing apparatus (for example, mouse or trackball), and the user may use the keyboard and the pointing apparatus to provide input to the computer. Other types of apparatuses may also be used to provide interaction with the user; for example, feedback provided to the user may be any form of sensory feedback (for example, visual feedback, auditory feedback, or tactile feedback); and any form (including acoustic input, voice input, or tactile input) may be used to receive input from the user.

The systems and technologies described herein may be implemented in a computing system that includes backend components (e.g., as a data server), or a computing system that includes middleware components (e.g., application server), or a computing system that includes frontend components (for example, a user computer having a graphical user interface or a web browser, through which the user may interact with the implementations of the systems and the technologies described herein), or a computing system that includes any combination of such backend components, middleware components, or frontend components. The components of the system may be interconnected by any form or medium of digital data communication (e.g., communication network). Examples of the communication network include: local area networks (LAN), wide area networks (WAN), the Internet, and blockchain networks.

The computer system may include a client and a server. The client and the server are generally far from each other and usually interact through the communication network. The relationship between the client and the server is generated by computer programs that run on the corresponding computer and have a client-server relationship with each other.

The technical solution of the present disclosure, by first determining a graph of undirected relationships between a plurality of subjects based on time characteristics of co-occurrences among the plurality of subjects; then acquiring time characteristic intervals of edges between an individual subject and related subjects thereof from the graph of the undirected relationships, where a time characteristic interval of an edge represents that the individual subject and a related subject co-occurred within the time characteristic interval; sorting the time characteristic intervals, and traversing the time characteristic intervals according to a sorted order, to find a target time characteristic interval not overlapping with a traversed time characteristic interval; and obtaining a directed one-way edge of the individual subject, by using a related subject corresponding to the target time characteristic interval as an end vertex of the one-way edge and using the individual subject as a start vertex of the one-way edge, so that it is possible to more conveniently and accurately trace the infection path of infected persons, thereby reducing the time for tracing the infection source.

It should be understood that the various forms of processes shown above may be used to reorder, add, or delete steps. For example, the steps described in the present disclosure may be performed in parallel, sequentially, or in different orders. As long as the desired results of the technical solution disclosed in the present disclosure can be achieved, no limitation is made herein.

The above specific embodiments do not constitute limitation on the protection scope of the present disclosure. Those skilled in the art should understand that various modifications, combinations, sub-combinations and substitutions may be made according to design requirements and other factors. Any modification, equivalent replacement and improvement made within the spirit and principle of the present disclosure shall be included in the protection scope of the present disclosure.

## Claims

1. A method for converting an undirected relationship to a directed relationship, comprising:
determining (201) a graph of undirected relationships between a plurality of subjects based on time characteristics of co-occurrences among the plurality of subj ects;
acquiring (202) time characteristic intervals of edges between an individual subject and related subjects thereof from the graph of the undirected relationships, wherein a time characteristic interval of an edge represents that the individual subject and a related subject co-occurred within the time characteristic interval;
sorting (203) the time characteristic intervals, and traversing the time characteristic intervals according to a sorted order, to find a target time characteristic interval not overlapping with a traversed time characteristic interval; and
obtaining (204) a directed one-way edge of the individual subject, by using a related subject corresponding to the target time characteristic interval as an end vertex of the one-way edge and using the individual subject as a start vertex of the one-way edge.

2. The method according to claim 1, further comprising:
merging (605) directed one-way edges of a plurality of individual subjects in the graph of the undirected relationships; and
removing (606) one-way edges having opposite directions between two individual subjects, to obtain a graph of one-way edges of the graph of the undirected relationships.

3. The method according to claim 2, further comprising:
in response to there being no one-way edge between an individual subject and a related subject thereof in the graph of the undirected relationships, marking an edge between the individual subject and the related subject thereof as a two-way edge, to obtain a graph of two-way edges of the graph of the undirected relationships.

4. The method according to claim 3, comprising:
determining a graph of directed edges between individual subjects and related subjects thereof in the graph of the undirected relationships, based on SPARK-GraphX by using an aggregate function of GraphX, wherein the graph of directed edges includes a one-way edge and a two-way edge.

5. The method according to claim 1, further comprising:
determining the time characteristics of the co-occurrences among the plurality of subjects based on the time of the earliest co-occurrence and the time of the most recent co-occurrence.

6. An apparatus (800) for converting an undirected relationship to a directed relationship, comprising:
a determination module (801), configured to determine (201) a graph of undirected relationships between a plurality of subjects based on time characteristics of co-occurrences among the plurality of subjects;
an acquisition module (802), configured to acquire (202) time characteristic intervals of edges between an individual subject and related subjects thereof from the graph of the undirected relationships, wherein a time characteristic interval of an edge represents that the individual subject and a related subject co-occurred within the time characteristic interval;
a searching module (803), configured to sort (203) the time characteristic intervals, and traversing the time characteristic intervals according to a sorted order, to find a target time characteristic interval not overlapping with a traversed time characteristic interval; and
a conversion module (804), configured to obtain (204) a directed one-way edge of the individual subject, by using a related subject corresponding to the target time characteristic interval as an end vertex of the one-way edge and using the individual subject as a start vertex of the one-way edge.

7. The apparatus according to claim 6, further comprising:
a merging module, configured to merge (605) directed one-way edges of a plurality of individual subjects in the graph of the undirected relationships;
a one-way graph module, configured to remove (606) one-way edges having opposite directions between two individual subjects, to obtain a graph of one-way edges of the graph of the undirected relationships.

8. The apparatus according to claim 7, further comprising:
a two-way graph module, configured to: mark an edge between the individual subject and the related subject thereof as a two-way edge, to obtain a graph of two-way edges of the graph of the undirected relationships, in response to there being no one-way edge between an individual subject and a related subject thereof in the graph of the undirected relationships.

9. The apparatus according to claim 6, wherein the apparatus is further configured to:
determine a graph of directed edges between individual subjects and related subjects thereof in the graph of the undirected relationships, based on SPARK-GraphX by using an aggregate function of GraphX, wherein the graph of directed edges includes a one-way edge and a two-way edge.

10. The apparatus according to claim 6, further comprising:
a relationship determination module, configured to determine the time characteristics of the co-occurrences among the plurality of subjects based on the time of the earliest co-occurrence and the time of the most recent co-occurrence.

11. An electronic device, comprising:
at least one processor; and
a memory, in communication with the at least one processor; wherein,
the memory stores instructions executable by the at least one processor to enable the at least one processor to perform the method according to any one of claims 1-5.

12. A non-transitory computer-readable storage medium having stored thereon computer instructions executable for causing the computer to perform the method according to any one of claims 1-5.

13. A computer program product comprising a computer program, the computer program, when executed by a processor (601), implementing the method according to any one of claims 1-5.
